# DEMANDE DE BREVET EUROPEEN

(11) **EP 0 875 221 A1**
(43) Date de publication de la demande: **04.11.1998**
(21) Numéro de dépôt: 98440047.3
(22) Date de dépôt: 11.03.1998
(51) Int. Cl.: A61F 11/00

(54) **Instrument de curetage à main et son procédé de fabrication**

(30) Priorité: 11.03.1997 FR 9703052
(71) Demandeur: Sittler, Jean-Pierre, 67230 Herbsheim (FR)
(72) Inventeur: Sittler, Jean-Pierre, 67230 Herbsheim (FR)
(74) Mandataire: Metz, Paul

(57) **Abrégé**

Le bâtonnet (2) présente au moins à l'une de ses extrémités un moyen de curetage en forme de bulbe (4) qui se compose d'une pluralité de lamelles (5) reliées entre elles au niveau d'une de leurs extrémités (8), et solidarisées par leur autre extrémité (11) au bâtonnet (2). Ces lamelles (5) présentent des chants dont les bords d'attaque sont conformés en arêtes.

Selon le procédé, on réalise par moulage-injection le bâtonnet (2) avec des extrémités en lanières (20), ces lanières présentent chacune une prolongation en forme de brin (21). Ces brins (21) sont utilisés pour réunir les extrémités des lanières et procéder à leur solidarisation.

Cette invention intéresse notamment les fabricants de bâtonnets cure-oreilles connus sous le nom de cotons-tiges.

## Description

La présente invention se rapporte à un instrument de curetage à main pour le nettoyage de conduits à utiliser par exemple pour l'hygiène du conduit auditif.

Elle se rapporte également au procédé de fabrication de cet instrument de curetage.

Pour évacuer la cire ou cérumen contenue dans le conduit auditif d'une oreille, on utilise habituellement un bâtonnet semi-rigide comportant des tampons de coton à chacune de ses extrémités. Ce bâtonnet est appelé communément "coton-tige".

Après avoir introduit le coton-tige dans le conduit auditif, on lui imprime un mouvement de rotation sur lui-même qui permet au tampon de coton de venir nettoyer par essuyage les parois du conduit.

Ces cotons-tiges ne donnent pas entière satisfaction pour cet usage. En effet, le tampon de coton se trouvant à l'extrémité du bâtonnet est de section axiale sensiblement ovoïde, donc avec un bout rond et relativement gros qui tend à repousser une partie du cérumen vers le fond du conduit auditif, pouvant provoquer la formation d'un bouchon.

On a essayé de remédier à cet inconvénient en conformant les tampons de coton selon un profil de forme sensiblement conique à pointe dirigée vers l'extérieur. L'introduction des cotons-tiges dans le conduit auditif à nettoyer devient ainsi plus facile. Cependant, si les tampons épousent bien les formes intérieures du conduit ils ne peuvent ni détacher ni extraire la cire incrustée sur les parois du conduit mais, au contraire, ne font que la repousser au fond de l'oreille.

Pour remédier à cet autre inconvénient, la publication de brevet français n° 2,600,883 enseigne un dispositif d'évacuation du cérumen constitué d'un élément extracteur solidaire d'une tige de manoeuvre. L'élément extracteur se présente sous la forme d'une pluralité de griffes reliées entre elles au niveau d'une de leurs extrémités et individualisées sur le restant de leur longueur. Ces griffes sont avantageusement réalisées en un matériau plastique souple, de sorte que la faible pression due au mouvement d'introduction de l'instrument de curetage dans le conduit auditif suffit à les déformer de façon élastique, leur permettant d'épouser les formes dudit conduit. Toutefois, par crainte des risques de blessures, les griffes sont conformées en tiges souples chacune à extrémité libre incurvée. Lors de la manoeuvre de rotation du cure-oreilles, les tiges vont ainsi simplement glisser sur les parois du conduit sans parvenir à détacher les dépôts de cérumen incrustés sur ces dernières.

Par ailleurs, l'effet de préhension n'est pas assuré car les dépôts de cire, même s'ils sont détachés, échappent à la prise en raison de la forme arrondie des extrémités des griffes et du caractère gras de ces dépôts. Seul un obstacle d'une certaine dimension pourra être saisi et extrait. Dans ces conditions, ce type d'instrument n'apporte aucune solution intéressante pour le nettoyage efficace des conduits auditifs.

La présente invention a pour but de remédier à ces inconvénients et à d'autres encore. A cet effet, l'invention se rapporte à un instrument de curetage du type cure-oreilles formé d'un bulbe de curetage solidaire de l'une au moins des extrémités d'un bâtonnet.

Conformément à la caractéristique principale de l'invention, le bulbe de curetage est constitué d'une pluralité de lamelles reliées entre elles au niveau d'une de leurs extrémités, leur autre extrémité étant solidarisée à l'une des extrémités du bâtonnet ou se confondant avec elle, ces lamelles présentant par ailleurs des chants dont les arêtes sont conformées en bords racleurs pour débarrasser par raclage la surface des parois du conduit des dépôts appliqués ou incrustés.

Les lamelles sont réalisées en un matériau élastique relativement souple. Ainsi, lorsque l'instrument de curetage selon la présente invention est introduit dans le conduit, la légère pression due à ce mouvement suffit à déformer les lamelles de façon élastique, celles-ci venant alors épouser parfaitement les formes du conduit à nettoyer. Cet effet s'amplifie lorsque le regroupement des extrémités des lamelles vient buter contre un obstacle ou une paroi transversale au conduit. Le mouvement de rotation sur lui-même imprimé au bâtonnet de curetage permet ensuite aux lamelles de venir racler par les arêtes des bords d'attaque de leurs chants les parois du conduit à nettoyer, et de détacher les dépôts qui adhèrent sur ses parois.

La forme en bulbe de lamelles souples individualisées permet d'obtenir un effet hélicoïdal de poussée des déchets vers la sortie du conduit.

Lorsque l'utilisateur retire l'instrument de curetage de son oreille tout en le tournant sur lui-même, les dépôts de cérumen détachés des parois par raclage puis amenés vers la sortie par l'effet du mouvement hélicoïdal sont entraînés vers l'extérieur.

Selon une autre caractéristique importante de l'invention, l'ensemble des lamelles présente un effet de torsade ou de vrille hélicoïdale ou semi-hélicoïdale ou une aptitude à cet effet lors de la rotation du bâtonnet. Cet effet d'ensemble en torsade ou en vrille permet de recueillir et d'extraire plus facilement les dépôts de cérumen préalablement détachés des parois du conduit auditif par raclage.

L'invention se rapporte également au procédé de fabrication de l'instrument de curetage dans lequel on réalise le bâtonnet et les lanières d'extrémité par moulage-injection, on prévoit des prolongations aux lanières sous forme de brins on réunit les extrémités des lanières par ces brins et on les solidarise entre elles.

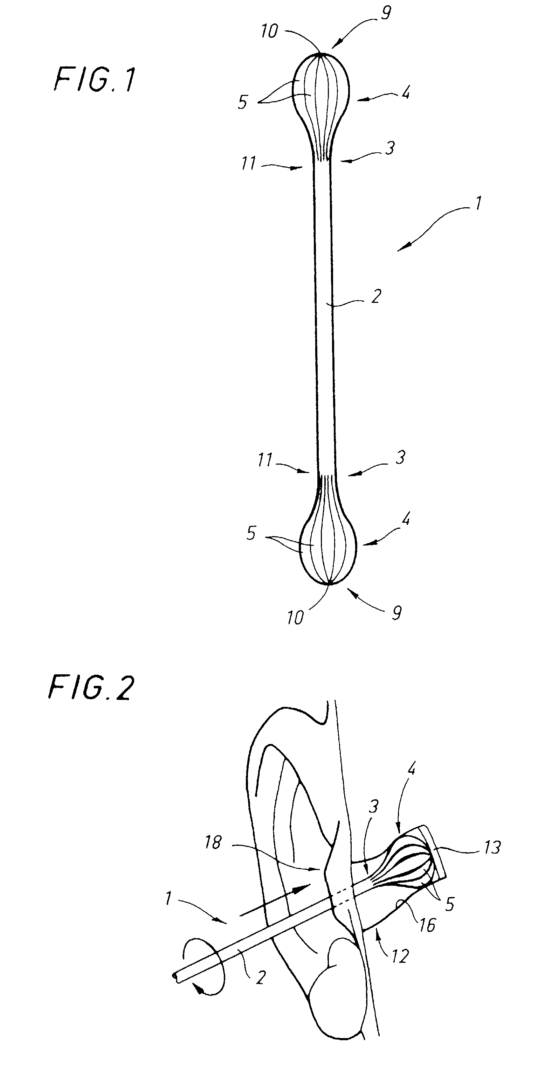

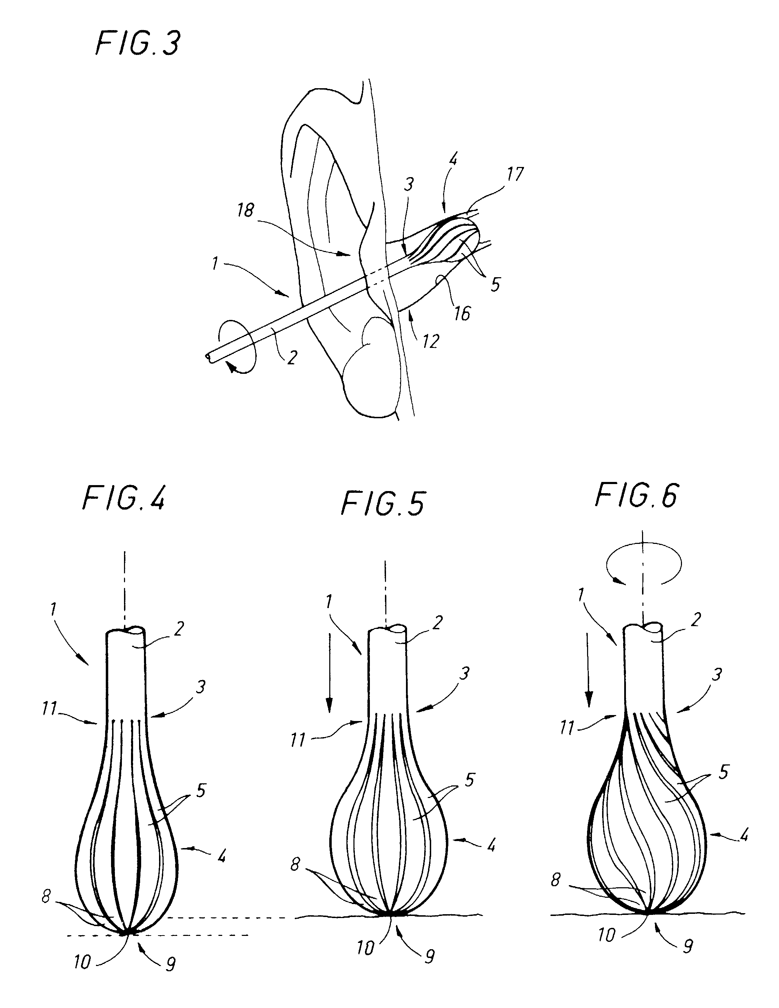

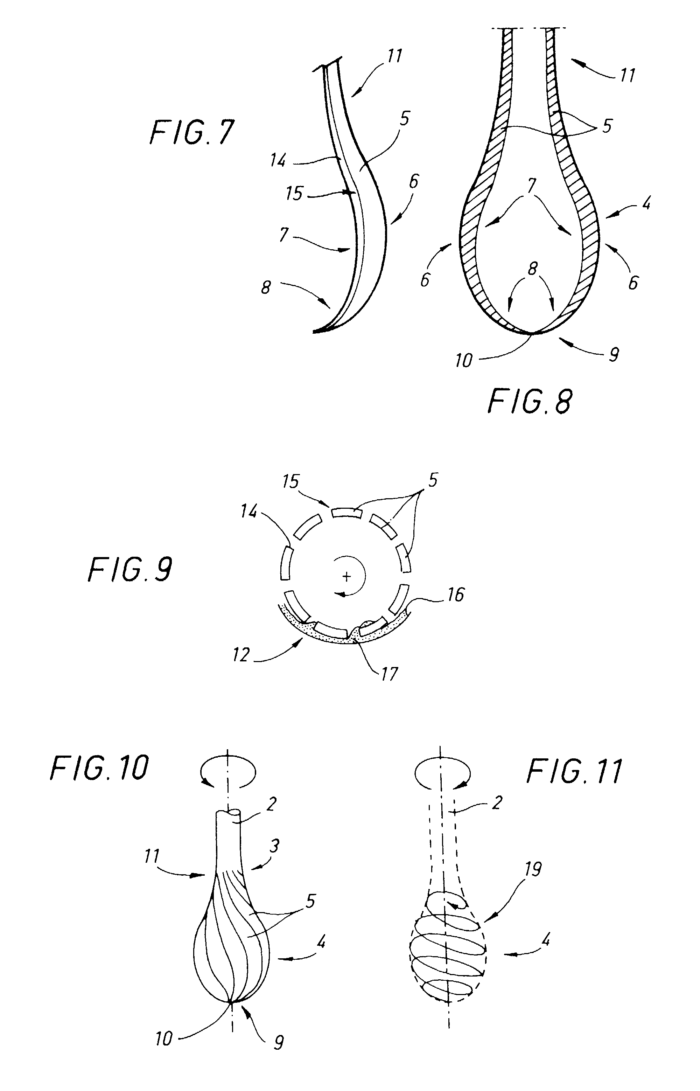

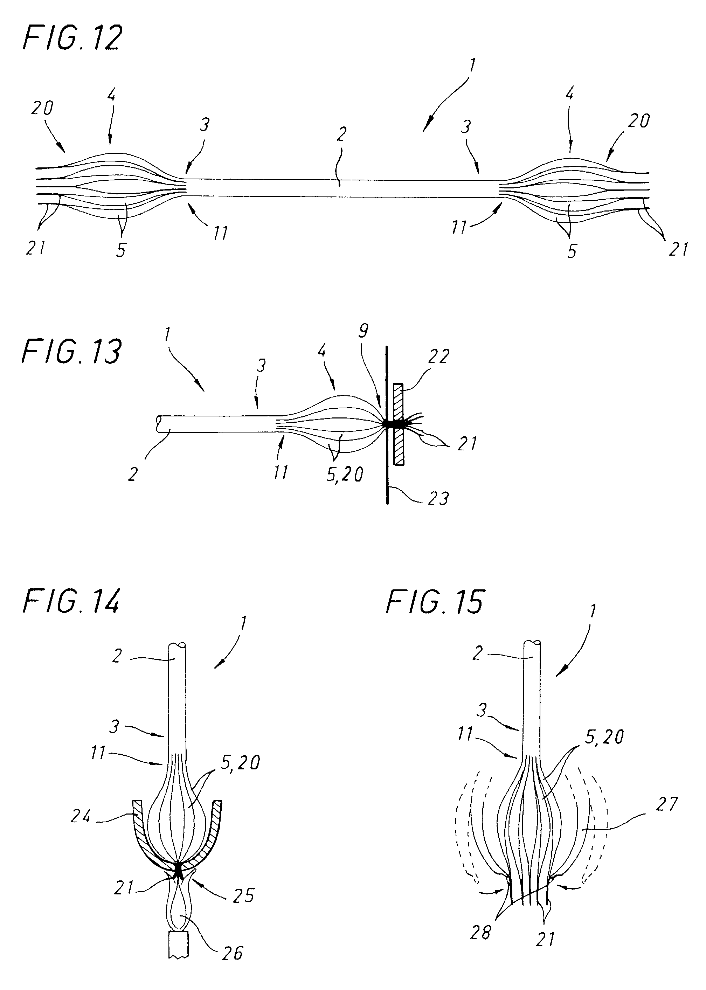

D'autres caractéristiques et avantages de la présente invention ressortiront mieux de la description qui suit effectuée à titre d'exemple selon un mode d'exécution préféré en référence au dessin accompagnant sur lequel :
. la figure 1 est une vue en perspective au repos de l'instrument de curetage selon l'invention ;
. la figure 2 est une vue en coupe transversale de l'instrument de curetage en butée au fond d'un conduit auditif ;
. la figure 3 est une vue en coupe transversale de l'instrument de curetage en cours de travail d'extraction dans un conduit auditif ;
. la figure 4 est une vue en perspective de l'extrémité de l'instrument de curetage au repos ;
. la figure 5 est une vue en perspective de l'extrémité du même instrument de curetage montrant l'effet de déformation des lamelles suite à un mouvement axial vers le bas et butée de l'extrémité du bulbe contre une paroi transversale ;
. la figure 6 est une vue en perspective de l'instrument de curetage en rotation sur lui-même lors du travail d'extraction dans un conduit ;
. la figure 7 est une vue en perspective d'une lamelle à l'état incurvé ;
. la figure 8 est une vue en coupe longitudinale du bulbe au niveau de deux lamelles en regard ;
. la figure 9 est une vue en coupe transversale du bulbe illustrant le travail de raclage des lamelles ;
. les figures 10 et 11 sont des vues schématiques montrant lors de la rotation, la déformation du bulbe et le tracé hélicoïdal d'extraction des déchets ;
. la figure 12 est une vue à plat du bâtonnet tel qu'il sort du moule d'injection ;
. la figure 13 est une vue schématique partiellement de dessus et partiellement en coupe d'une extrémité du bâtonnet lors de la phase de soudage et de sectionnement des brins d'extrémité ;
. la figure 14 est une vue schématique de la même phase en utilisant un conformateur à fond chauffant du type coupelle ;
. la figure 15 est une vue schématique en coupe de la même phase en utilisant un outil de préhension du type pince.

La présente invention procède de l'idée générale inventive qui consiste à réaliser l'extrémité de travail d'un instrument de curetage à main sous la forme d'un bulbe déformable constitué de la succession d'une pluralité de lamelles ou de lanières souples individualisées réunies entre elles par l'une de leurs extrémités et solidarisées individuellement directement ou indirectement à l'extrémité d'un bâtonnet par l'autre de leurs extrémités ou constituant la prolongation individualisée en lanières de la matière dans laquelle est réalisé le corps du bâtonnet. Plus précisément, les lamelles ou lanières sont plates et présentent des chants à bords d'attaque racleurs et une forme de profil de préférence légèrement incurvé pour favoriser la déformation en écrasement du bulbe en fond de conduit lorsqu'il se trouve en appui de butée sur un front transversal au conduit.

Une des applications principales de cet instrument de curetage concerne le nettoyage des conduits auditifs, mais bien d'autres applications de curetage de conduits ou analogues peuvent être envisagées.

L'extrémité en bulbe permet ainsi d'épouser les formes du conduit auditif sous l'effet de la légère pression due à l'introduction de l'instrument de curetage dans le conduit auditif, et à détacher ensuite par raclage les dépôts par exemple de cire qui adhèrent aux parois. Les lamelles présentent en plus de leurs bords racleurs un effet de torsade ou de vrille ou une aptitude à cet effet lors de la rotation de l'instrument de curetage sur lui-même, ce qui permet dans le même mouvement de détacher par raclage puis d'amener vers la sortie par poussée les dépôts détachés des parois.

Le caractère souple et la constitution du bulbe permettent à celui-ci de s'expanser au moindre contact d'obstruction dans le conduit à nettoyer. On assure ainsi un amortissement du choc et un ralentissement puis un arrêt de la progression du bâtonnet.

L'instrument de curetage selon l'invention, désigné dans son ensemble par la référence 1, est constitué d'un bâtonnet 2 à au moins une des extrémités 3 duquel est soit solidarisé, par collage, thermosoudage ou autre technique de solidarisation, soit conformé dans la masse, un moyen de curetage sous la forme d'un bulbe 4.

Conformément à la caractéristique principale de l'invention, le moyen de curetage conformé en bulbe 4 est constitué d'une pluralité de lamelles ou de lanières individuelles telles que 5 réalisées en un matériau élastique, plastique ou analogue présentant tous des propriétés mécaniques adaptées conférant au bulbe 4 formé de la juxtaposition circulaire des lamelles ou lanières un caractère aisément déformable tout en gardant à chacune des lamelles 5 une rigidité minimale et suffisante pour assurer un travail de raclage efficace. Chacune des lamelles 5 présente dans sa zone médiane 6 une forme incurvée convexe 7. Les lamelles ou lanières sont réunies entre elles par l'une de leurs extrémités 8 en une zone de regroupement 9 correspondant à l'extrémité 10 du bulbe. Les lamelles 5 sont solidarisées individuellement directement ou indirectement par exemple au moyen d'une pièce de regroupement (non représentée) par leur autre extrémité 11 à l'extrémité 3 du bâtonnet 2 ou sont conformées dans la masse du corps du bâtonnet par un procédé industriel de formage par exemple par moulage-injection.

Sous l'effet de la légère pression due à l'introduction du bâtonnet 2 dans un conduit 12 à nettoyer, les lamelles 5 se déforment de façon élastique, ce qui leur permet d'épouser parfaitement les formes intérieures du conduit 12 par exemple auditif.

Lorsque l'extrémité du bulbe 4 arrive en contact avec un obstacle 13 transversal au conduit 12, par exemple le tympan, son écrasement sous l'effet de la poussée axiale contre l'obstacle provoque son expansion.

Les lamelles 5 présentent des chants 14 dont les bords d'attaque sont conformés en arêtes vives non-blessantes telles que 15. Ainsi, le mouvement de rotation sur lui-même procuré au bâtonnet 2 permet aux lamelles 5 de venir détacher par raclage des parois 16 du conduit puis emporter par poussée extractive les dépôts 17 par exemple de cire vers la sortie 18 du conduit.

Après introduction du bâtonnet 2 et rotation de celui-ci sur lui-même pour détacher les dépôts 17 qui adhèrent aux parois, l'utilisateur va retirer l'instrument de curetage du conduit 12 en poursuivant la rotation. Cette action permet d'extraire les déchets 17 hors du conduit 12 après les avoir amassés à la périphérie du conduit. En effet, la rotation du bâtonnet 2 combinée à la translation de sortie provoquera la sortie de l'extrémité en bulbe 4 avec les lamelles 5 en torsade ou en vrille. L'ensemble de ces mouvements provoque par entraînement hélicoïdal selon une hélice 19 la remontée des dépôts jusqu'à la sortie 18 du conduit 12.

Selon une variante, les lamelles 5 sont suffisamment souples pour présenter naturellement une aptitude à l'effet de torsade ou de vrille lors de l'extraction du cure-oreilles 1 selon un mouvement hélicoïdal ou semi-hélicoïdal.

L'invention se rapporte également au procédé de fabrication de l'instrument de curetage décrit ci-dessus.

Le procédé de fabrication de l'invention procède de l'idée générale inventive qui consiste à réaliser le bâtonnet par un procédé de moulage par injection en prévoyant des prolongations aux lanières sous la forme de brins et d'utiliser ces brins pour réaliser la réunion d'extrémité des lanières avec immobilisation par soudure.

Selon ce procédé, on réalise par moulage-injection des bâtonnets 2 en matière plastique présentant chacun à chacune de leurs extrémités une série de lanières 20, par exemple en nombre de huit à dix, régulièrement réparties sur la circonférence de l'extrémité dont elles constituent la prolongation. Les lanières 20 sont donc formées d'une seule pièce dans la masse du bâtonnet. Elles sont disposées et orientées de manière à présenter leur face arrière vers la partie centrale d'extrémité traversée par l'axe du bâtonnet 2.

Les lanières 20 ont ceci de particulier qu'elles présentent chacune une prolongation d'extrémité de plus faible largeur sur une certaine longueur par exemple sous la forme de brins tels que 21.

Ces prolongations 21 serviront au regroupement et au maintien pendant le traitement thermique de soudage que l'on décrira ci-après.

Les extrémités des bâtonnets sortent du moule d'injection dans les formes et caractéristiques techniques représentées sur la figure 12.

Après la sortie du moule, les bâtonnets sont rangés et posés sur un support individuel ou un support collectif à emplacements individuels et maintenus en vue du groupement des extrémités libres des lanières et de leur immobilisation par soudage.

Un dispositif de préhension viendra grouper les lanières et les immobiliser groupées par leurs extrémités pour former un faisceau convergent avec les brins d'extrémité en les maintenant serrés par leurs extrémités.

On peut utiliser à cet effet une bague convergente de serrage 22 comme représenté sur la figure 13.

Les brins étant immobilisés, on sectionne par un instrument de coupe chauffant 23 le cordon formé par la réunion des brins. Il reste un petit appendice que l'on peut presser à chaud ou éliminer par sectionnement, abrasion ou tout autre moyen.

Comme déjà indiqué, les lanières sont plus ou moins souples et on peut leur conférer dès le moulage une forme légèrement incurvée.

En alternative, on peut réaliser des bâtonnets 2 plus longs que l'on sectionnera en deux demi bâtonnets ou réaliser de suite un bâtonnet à une seule extrémité de curetage.

Ces bâtonnets seront alors présentés en position verticale et engagés dans une forme permettant le regroupement et le pincement-maintien des brins. On procède ensuite à un rapprochement pour obtenir un certain écrasement du bulbe 4 tout en réalisant une fusion-soudage pour réunir les extrémités des lanières.

Une manière particulière de procéder consiste à présenter ces bâtonnets en position verticale et à les engager dans une forme conique ou en cuvette 24 à fond chauffant 25 fermé ou traversant, en contact ou non avec une flamme 26 pour arriver à la fusion-soudage des brins (figure 14). On peut dans cette variante torsader les brins par un mouvement de rotation du bâtonnet 2.

On peut envisager également d'utiliser un instrument de préhension en coupelle ouvrable 27 selon un plan médian destiné à venir pincer les brins par rapprochement de ses bords 28 (figure 15). Il suffit de prévoir des bords chauffants pour réaliser la soudure des brins entre eux et par conséquent celle des extrémités des lanières avec détachement de la queue des brins par sectionnement.

Il va de soi que l'invention n'est pas limitée dans ses applications au nettoyage des conduits auditifs et que diverses autres applications, variantes et modifications simples entrent dans son cadre.

Il en est de même pour le procédé qui peut subir quelques variations tout en restant dans le cadre de l'invention.

## Revendications

1. Instrument de curetage à main formé d'un bâtonnet (2) et d'au moins un moyen de curetage solidaire de l'une des extrémités (3) du bâtonnet (2), caractérisé en ce que le moyen de curetage est constitué d'une pluralité de lamelles ou lanières souples (5) individualisées, solidarisées entre elles au niveau d'une de leurs extrémités libres (8) et solidarisées par leur autre extrémité (11) à l'extrémité (3) du bâtonnet (2) ou réalisées dans la matière même du bâtonnet constituant à cette extrémité du bâtonnet une forme bombée en bulbe (4) susceptible de déformation lorsque le bulbe (4) est en contact frontal de butée.

2. Instrument selon la revendication 1, caractérisé en ce que les lamelles (5) sont plates et présentent des bordures de chants (14) conformées en arêtes (15) par lesquelles elles viennent racler les parois du conduit à nettoyer.

3. Instrument de curetage selon l'une des revendications 1 ou 2, caractérisé en ce que les lamelles (5) présentent lors de la rotation du bâtonnet (2) sur lui-même un effet de torsade ou de vrille permettant une extraction par entraînement hélicoïdal des dépôts désincrustés

4. Instrument de curetage selon l'une quelconque des revendications précédentes de 1 à 3, caractérisé en ce que les lamelles (5) sont solidarisées individuellement sur l'extrémité du bâtonnet.

5. Instrument de curetage selon l'une quelconque des revendications précédentes de 1 à 3, caractérisé en ce que les lamelles (5) sont regroupées avant d'être solidarisées sur l'extrémité du bâtonnet (2).

6. Procédé de fabrication d'un instrument de curetage du type bâtonnet (2) tel que revendiqué ci-dessus caractérisé en ce que l'on forme par moulage-injection le corps du bâtonnet avec deux extrémités en une pluralité de lanières (20) et en ce que chacune de ces lanières présentent une prolongation en forme de brin (21), les brins étant utilisés pour réunir les extrémités des lanières en vue de leur solidarisation.

7. Procédé selon la revendication 6, caractérisé en ce que l'on regroupe les brins et on les maintient réunis.

8. Procédé selon la revendication 6 ou 7, caractérisé en ce que l'on sectionne les brins (21) regroupés et on les soude simultanément par un outil tranchant et chauffant (23).

9. Procédé selon la revendication 6 ou 7, caractérisé en ce que l'on regroupe les brins (21) et on les maintient puis on écrase légèrement le bulbe (4) tout en procédant à une fusion-soudage.

10. Procédé selon la revendication 6 ou 7, caractérisé en ce que l'on regroupe les brins (21) dans un réceptacle conique ou en cuvette (24) à fond chauffant (25) ou par un outil de type pince (27) à bords chauffants (28), le bâtonnet (2) étant en position verticale et en ce que l'on réalise la réunion des extrémités des lanières par fusion-soudage, le cas échéant avec sectionnement par les bords chauffants (28) de la pince.
